# EUROPEAN PATENT APPLICATION

(11) **EP 4 349 349 A1**
(43) Date of publication of application: **10.04.2024**
(21) Application number: 22867190.5
(22) Date of filing: 23.08.2022
(51) Int. Cl.: A61K 35/33, A61P 13/12

(54) **FIBROBLAST-CONTAINING PHARMACEUTICAL COMPOSITION FOR TREATING KIDNEY DISEASE**

(30) Priority: 08.09.2021 JP 2021146130
(71) Applicant: Metcela Inc., Kanagawa 2100821 (JP)
(72) Inventor: IWAMIYA, Takahiro, Tsuruoka-shi, Yamagata 997-0002 (JP); HOMMA, Jun, Tokyo 162-8666 (JP); IMAMURA, Tomomi, Tsuruoka-shi, Yamagata 997-0002 (JP); MATSUOKA, Yuimi, Tsuruoka-shi, Yamagata 997-0002 (JP)
(74) Representative: Jones, Nicholas Andrew
(86) International application number: PCT/JP2022/031721
(87) International publication number: WO 2023/037868

(57) **Abstract**

Based on the discovery that fibroblasts suppress renal fibrosis to recover the renal function in a rat model with a kidney disease, a pharmaceutical composition for treatment of a kidney disease, the composition including fibroblasts, was successfully provided.

## Description

### TECHNICAL FIELD

The present invention relates to a pharmaceutical composition for treatment of a kidney disease, comprising fibroblasts.

### BACKGROUND ART

The total number of patients with acute kidney injury (AKI), chronic kidney disease (CKD), and renal replacement therapy (RRT) has exceeded 850 million. Due to aging and an increased prevalence of lifestyle-related diseases, there is a trend towards an increase in the number of patients, and this has become a public health problem in recent years.
AKI is a disease in which the glomerular filtration rate decreases rapidly and progressively after injury, leading to a decrease in the renal function (Non-Patent Document 1). Even in cases where patients are receiving clinical treatment for AKI, some of the patients develop progressive CKD. CKD is a disease in which the kidneys are damaged or become incapable of filtrating the blood like healthy kidneys. In cases where CKD is left untreated, renal fibrosis gradually progresses, resulting in necrosis of nephrons in a wide area, and hence a decrease in the filtration capacity of the kidneys. In cases where the renal function is decreased to not more than 30% of the normal function (that is, where the CKD severity classification is below G4 (the CKD severity classification includes the groups G1 to G5, wherein G1 represents the normal renal function, and G5 represents end-stage renal failure)), dialysis or RRT is required. Dialysis is a therapeutic method for the purpose of partially supporting the renal function of a patient, but not for the purpose of recovery of the renal function. Dialysis requires regular outpatient treatment. Furthermore, long-term dialysis treatment may lead to the requirement of additional partial treatment for complications associated with the dialysis treatment. Further, RRT, which is the only curative treatment, is not applicable to all dialysis patients due to the overwhelming shortage of donors. Because of such a background, development of novel therapeutic methods has been demanded not only from the viewpoint of improving the quality of life (QOL) of the patients, but also from the viewpoint of reducing the heavy burden on social security.

In order to address this clinical problem, various strategies have been developed. Examples of such strategies include cell therapies using mesenchymal stem cells (MSCs) (Non-Patent Documents 2 and 3) or pluripotent stem cells (Non-Patent Document 4). However, the effects of these methods on the functional recovery have been limited.

In recent years, renal lymphangiogenesis has attracted attention not only because it maintains the homeostasis of the body fluid of the kidney after damaging, but also because it transports immune cells and antigens, leading to reduction of renal fibrosis (Non-Patent Document 5). Renal fibrosis is thought to play a central role in the progression of renal failure. Therefore, in order to slow the progression of chronic renal failure, antihypertensive drugs that suppress the development of fibrosis are often used.

The present inventors have reported that addition of a cocktail of TNF-α and IL-4 promotes expression of VCAM1 (CD106) and Thy-1 (CD90) in cardiac fibroblasts (CFs) (Patent Document 1).

### PRIOR ART DOCUMENTS

### [Patent Document]

[Patent Document 1] WO 2020/045547

### [Non-patent Documents]

[Non-Patent Document 1] J. Am. Soc. Nephrol. 16, 3149-3150 (2005)
[Non-Patent Document 2] Curr. Opin. Nephrol. Hypertens. 25, 372-377 (2016)
[Non-Patent Document 3] Stem Cell Res. Ther. 5, 83 (2014)
[Non-Patent Document 4] Cell Stem Cell 21, 730-746.e6 (2017)
[Non-Patent Document 5] Nat. Rev. Nephrol. 16, 289-303 (2020)

### SUMMARY OF THE INVENTION

### PROBLEMS TO BE SOLVED BY THE INVENTION

An object of the present invention is to provide a pharmaceutical composition for treatment of a kidney disease, comprising fibroblasts.

### MEANS FOR SOLVING THE PROBLEMS

In order to solve the above problem, the present inventors studied to discover that fibroblasts suppress renal fibrosis to recover the renal function in a rat model with a kidney disease, thereby reaching the present invention.

More specifically, the present invention includes the following first aspect (Invention A).
(A1) A pharmaceutical composition for treatment of a kidney disease, the composition comprising VCAM1⁺ and CD90⁺ fibroblasts,
   wherein the ratio of the VCAM1⁺ and CD90⁺ fibroblasts in the total fibroblasts in the pharmaceutical composition is more than 1.08%.
(A2) The pharmaceutical composition according to (A1), wherein the fibroblasts are cardiac fibroblasts.
(A3) The pharmaceutical composition according to (A1) or (A2), wherein the fibroblasts are derived from an adult.
(A4) The pharmaceutical composition according to any one of (A1) to (A3), wherein the kidney disease is acute kidney injury or chronic kidney disease.
(A5) The pharmaceutical composition according to any one of (A1) to (A4), which is an injectable composition.
(A6) The pharmaceutical composition according to any one of (A1) to (A4), wherein the fibroblasts are constructed as a planar or three-dimensional cellular tissue.
(A7) A therapeutic agent for a kidney disease, the agent comprising cardiac fibroblasts.
(A8) The therapeutic agent according to (A7),
   wherein the cardiac fibroblasts comprise VCAM1⁺ and CD90⁺ fibroblasts, and
   wherein the ratio of the VCAM1⁺ and CD90⁺ fibroblasts in the total fibroblasts in the therapeutic agent is more than 1.08%.
(A9) The therapeutic agent according to (A7) or (A8), wherein the cardiac fibroblasts are derived from an adult.
(A10) The therapeutic agent according to any one of (A7) to (A9), wherein the kidney disease is acute kidney injury or chronic kidney disease.
(A11) The therapeutic agent according to any one of (A7) to (A10), which is an injectable composition.
(A12) The therapeutic agent according to any one of (A7) to (A10), wherein the fibroblasts are constructed as a planar or three-dimensional cellular tissue.

The present invention also includes the following second aspect (Invention B).
(B1) A method of treating a kidney disease in a subject, the method comprising administering or implanting a pharmaceutical composition comprising VCAM1⁺ and CD90⁺ fibroblasts to the subject.

The present invention also includes the following third aspect (Invention C).
(C1) Use of a pharmaceutical composition comprising VCAM1⁺ and CD90⁺ fibroblasts as an injection solution for administration to a kidney tissue and/or a surrounding area thereof, for treatment of a kidney disease.
(C2) Use of a pharmaceutical composition comprising VCAM1⁺ and CD90⁺ fibroblasts as an implant for transplantation to a kidney tissue and/or a surrounding area thereof, for treatment of a kidney disease.

### EFFECT OF THE INVENTION

By the present invention, a pharmaceutical composition for treatment of a kidney disease, the composition comprising fibroblasts, was successfully provided.

### BRIEF DESCRIPTION OF THE DRAWINGS

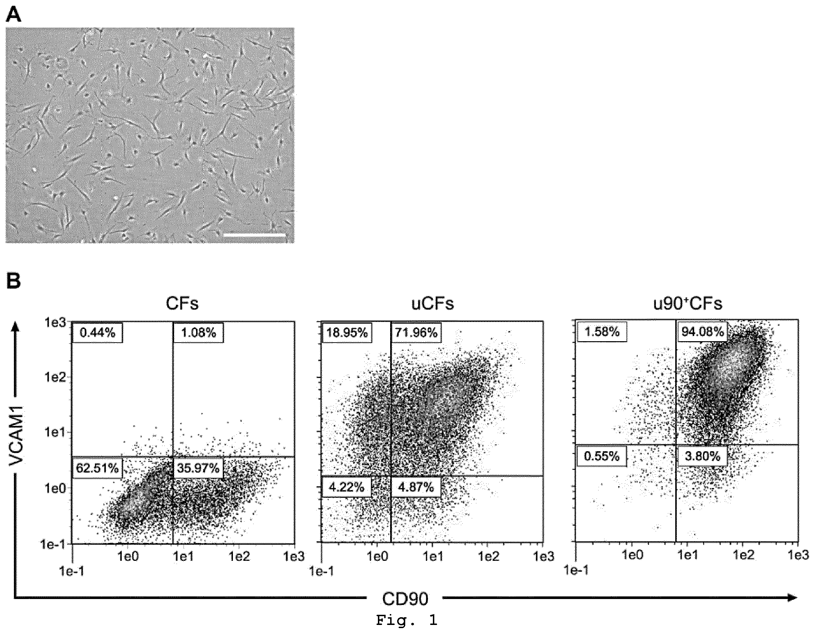

Fig. 1 is a diagram showing characteristics of cardiac fibroblasts (CFs). Fig. 1A shows a micrograph of primary cultured cardiac fibroblasts (scale bar: 500 µm). Fig. 1B is a diagram showing results of flow cytometry (FCM) analysis of CD90- and VCAM1-positive cells (%) in CFs, uCFs, and u90⁺ CFs.

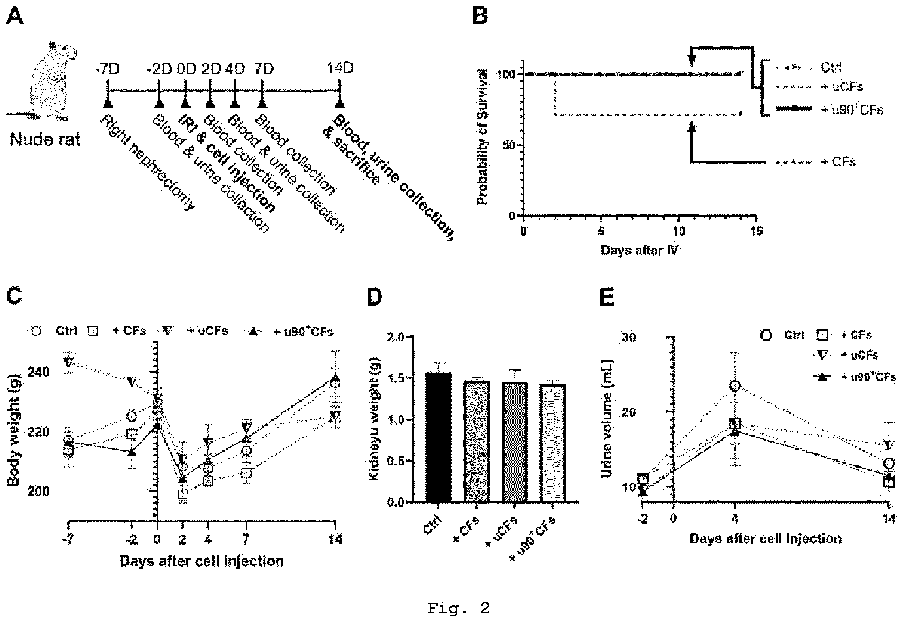

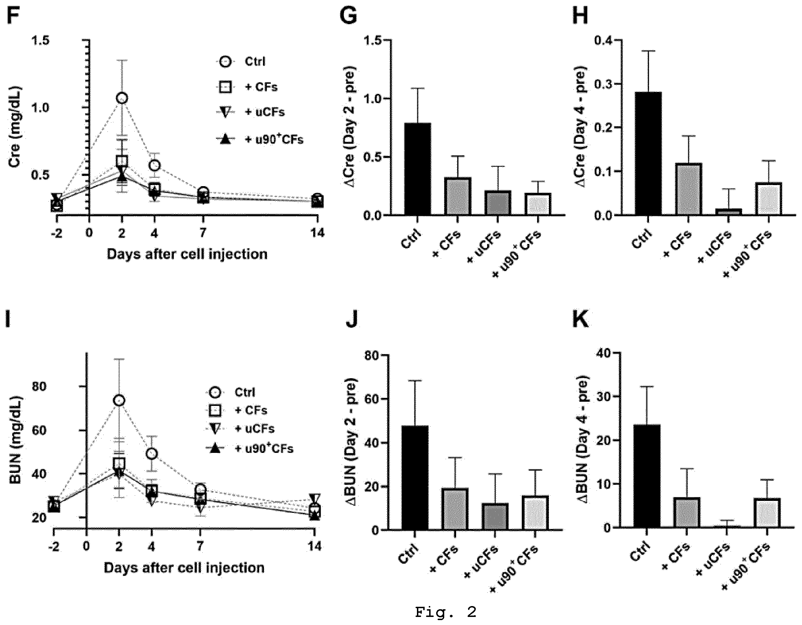

Fig. 2 is a diagram showing the survival rate, urine evaluation, and renal function evaluation of various cell administration groups. Fig. 2A shows an experimental design for preparation of an IRI (ischemia-reperfusion injury) rat model and cell administration. Nude rats on Day 7 after right nephrectomy (represented as 0D in the figure) were subjected to IRI treatment, and CFs, uCFs, u90⁺ CFs or a vehicle was/were administered immediately beneath the renal capsule in the rats. Fig. 2B shows the survival rate of each group after IRI. The groups are a vehicle control group (represented as Ctrl in the figure; n=7), a CF administration group (represented as +CFs in the figure; n=7), a uCF administration group (represented as +uCFs in the figure; n=2), and a u90⁺ CF administration group (represented as +u90⁺ CFs in the figure; n=4), respectively. Fig. 2C is a graph showing the body weight at each monitoring point (mean ± standard error (SE); Ctrl: n=7, +CFs: n=5, +uCFs: n=2, and +u90⁺ CFs: n=4). Fig. 2D is a graph showing the mean kidney weight on Day 14 after the cell administration (Ctrl: n=7, +CFs: n=5, +uCFs: n=2, and +u90⁺ CFs: n=4). Fig. 2E is a graph showing the urine output in each group at each monitoring point (mean ± standard error (SE); Ctrl: n=7, +CFs: n=5, +uCFs: n=2, and +u90⁺ CFs: n=4). Figs. 2F and 2I are graphs showing the serum creatinine level (Cre) and the blood urea nitrogen level (BUN) at each monitoring point (mean ± standard error (SE); Ctrl: n=7, +CFs: n=5, +uCFs: n=2, and +u90⁺ CFs: n=4). Figs. 2G, 2H, 2J, and 2K are graphs showing the elevation levels of Cre and BUN on Day 2 and Day 4 after the cell administration (mean ± standard error (SE); Ctrl: n=7, +CFs: n=5, +uCFs: n=2, and +u90⁺ CFs: n=4).

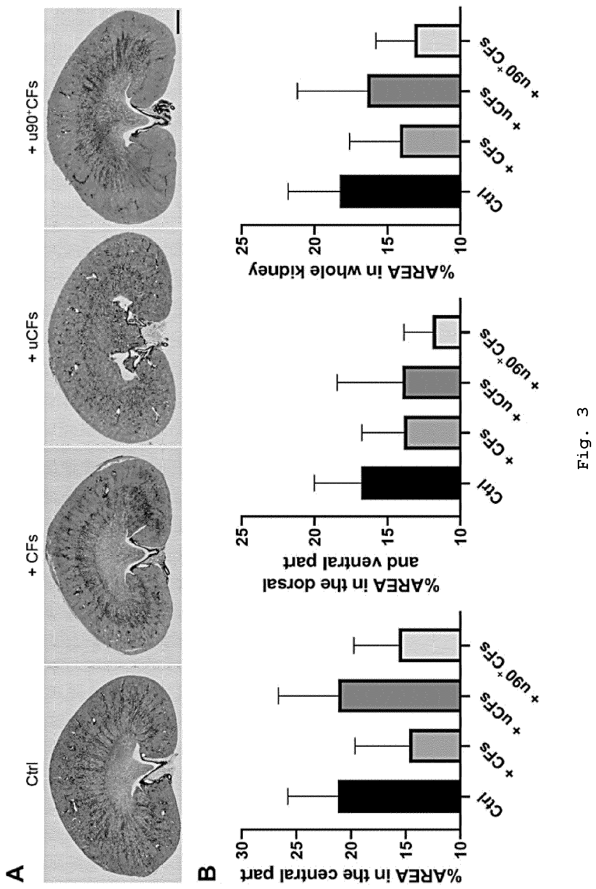

Fig. 3 is a diagram showing fibrosis-suppressing effects by administration of various fibroblasts. Fig. 3A shows micrographs of kidney sections on Day 14 after cell administration as observed by Sirius Red (SR) staining (scale bar: 2mm). Fig. 3B shows graphs obtained by quantification of the mean fibrotic area ratio on Day 14 after cell administration (Ctrl: n=7, +CFs: n=5, +uCFs: n=2, and +u90⁺ CFs: n=4). The quantification was carried out after dividing the kidney area into the three parts: the central part, the dorsal and ventral part, and the whole kidney.

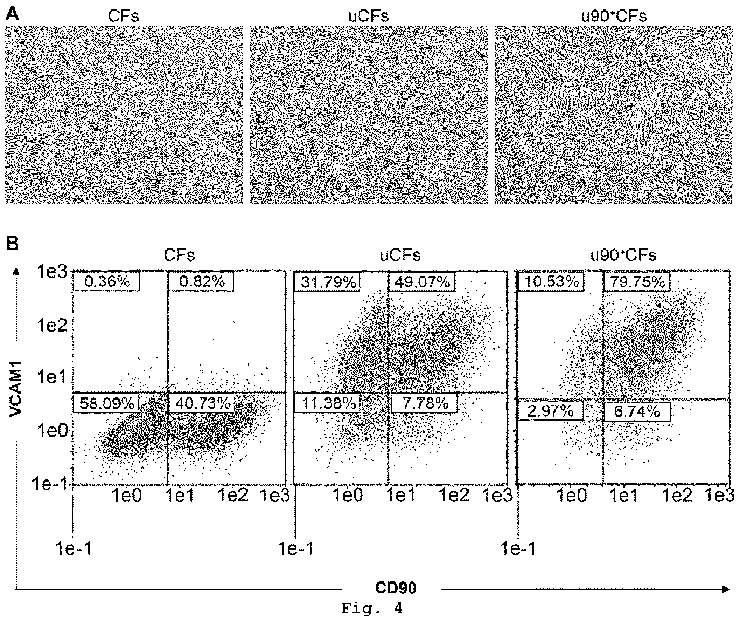

Fig. 4 is a diagram showing characteristics of the cardiac fibroblasts used in Example 3. Fig. 4A shows micrographs of various primary cultured cardiac fibroblasts (CFs, uCFs, and u90⁺ CFs). Fig. 4B is a diagram showing results of flow cytometry (FCM) analysis of CD90- and VCAM1-positive cells (%) in the CFs, uCFs, and u90⁺ CFs.

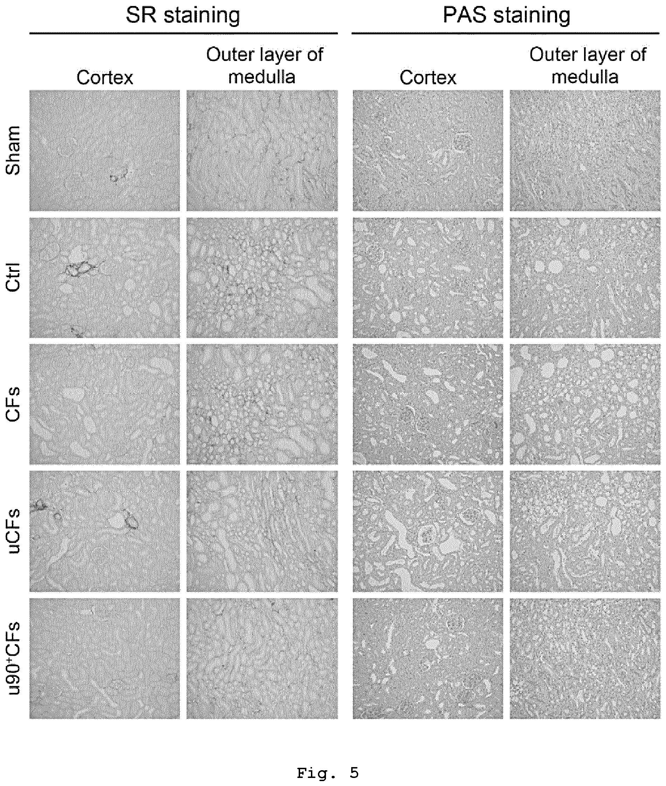

Fig. 5 shows micrographs of kidney sections (the cortical region and the medullary outer layer region) 1 week after administration of various fibroblasts (CFs, uCFs, and u90⁺ CFs) as observed by SR staining or PAS staining. In the figure, Ctrl shows stained images of a vehicle control group, and Sham shows stained images of left kidney tissues without ureteral ligation.

### MODE FOR CARRYING OUT THE INVENTION

The present invention is described below in detail by way of specific embodiments. However, the present invention is not limited to the specific embodiments described.

One embodiment of the present invention is a pharmaceutical composition for treatment of a kidney disease, the composition comprising VCAM1⁺ and CD90⁺ fibroblasts.

The origin of the fibroblasts is not limited. The fibroblasts used may be obtained by differentiation from pluripotent stem cells such as embryonic stem cells (ES cells), induced pluripotent stem cells (iPS cells), or Muse cells, or from adult stem cells such as mesenchymal stem cells. Further, primary cells collected from an animal (including a human) may be used, or a cell line may be used.

Examples of the fibroblasts include those derived from heart, kidney, or skin. The fibroblasts are preferably cardiac fibroblasts.

The fibroblasts are preferably, but do not need to be, derived from an adult.

The fibroblasts from which the VCAM1⁺ and CD90⁺ fibroblasts are derived may be originally expressing VCAM1 and/or CD90 at a certain level(s), or may not be expressing these at all.

The fibroblasts in the present embodiment may be fibroblasts expressing the cell surface markers that are normally expressed by normal fibroblasts, or may be fibroblasts in which an additional marker(s) is/are expressed or in which part of the markers are not expressed due to the later-described cell production method of the present invention.

In human-derived fibroblasts, the ratio of VCAM1⁺ and CD90⁺ fibroblasts is low. In cases of cardiac fibroblasts, the ratio is at most about 1.08% (in terms of the cell number) in the natural state. For example, although the ratio of VCAM1⁺ and CD90⁺ fibroblasts to the total fibroblasts contained in the pharmaceutical composition of the present embodiment is not limited, the ratio may be, for example, not less than 0.82%, may be not less than 1.08%, may be not less than 5%, may be not less than 10%, may be not less than 20%, may be not less than 30%, may be not less than 40%, may be not less than 49.07%, may be not less than 50%, may be not less than 60%, may be not less than 70%, may be not less than 71.96%, may be not less than 79.75%, may be not less than 80%, may be not less than 89.89%, may be not less than 90%, or may be not less than 95%; or may be more than 0.82%, may be more than 1.08%, may be more than 5%, may be more than 10%, may be more than 20%, may be more than 30%, may be more than 40%, may be more than 49.07%, may be more than 50%, may be more than 60%, may be more than 70%, may be more than 71.96%, may be more than 79.75%, may be more than 80%, may be more than 89.89%, may be more than 90%, or may be more than 95%; in terms of the cell number.

Although the ratio of VCAM1⁺ fibroblasts in the human-derived fibroblasts is not limited, the ratio may be, for example, not less than 1.18%, may be not less than 1.53%, may be not less than 5%, may be not less than 10%, may be not less than 20%, may be not less than 30%, may be not less than 40%, may be not less than 50%, may be not less than 60%, may be not less than 70%, may be not less than 80%, may be not less than 80.86%, may be not less than 90%, may be not less than 90.28%, may be not less than 90.91%, may be not less than 93.49%, or may be not less than 95%; or may be more than or not less than 1.18, may be more than 1.53%, may be more than 5%, may be more than 10%, may be more than 20%, may be more than 30%, may be more than 40%, may be more than 50%, may be more than 60%, may be more than 70%, may be more than 80%, may be more than 80.86%, may be more than 90%, may be more than 90.28%, may be more than 90.91%, may be more than 93.49%, or may be more than 95%; in terms of the cell number.

Although the ratio of CD90⁺ fibroblasts in the human-derived fibroblasts is not limited, the ratio may be, for example, not less than 41.55%, may be not less than 50%, may be not less than 55.17%, may be not less than 56.83%, may be not less than 60%, may be not less than 70%, may be not less than 76.83%, may be not less than 80%, may be not less than 86.49%, may be not less than 90%, may be not less than 94.58%, or may be not less than 95%; or may be more than 41.55%, may be more than 50%, may be more than 55.17%, may be more than 56.83%, may be more than 60%, may be more than 70%, may be more than 76.83%, may be more than 80%, may be more than 86.49%, may be more than 90%, may be more than 94.58%, or may be more than 95%, in terms of the cell number.

The fibroblasts in the pharmaceutical composition of the present embodiment may be a fibroblast population comprising the VCAM1⁺ and CD90⁺ fibroblasts described above.

### (Preparation of Fibroblasts)

As described above, in the preparation of the fibroblasts, the origin of the fibroblasts is not limited. The fibroblasts may be autologous cells. For example, cardiac fibroblasts isolated from a cardiac tissue of a patient with a cardiac disease, or cardiac fibroblasts isolated after allowing differentiation of adult (somatic) stem cells of a patient, are prepared. The fibroblasts may also be fibroblasts collected after allowing differentiation of pluripotent stem cells such as iPS cells derived from autologous cells. The fibroblasts may also be non-autologous cells. For example, a cardiac tissue derived from a donor who provides cardiac cells, cardiac fibroblasts isolated from a cardiac tissue prepared using an animal or the like, or cardiac fibroblasts isolated after allowing differentiation of adult (somatic) stem cells of a donor, is/are prepared. The fibroblasts may also be fibroblasts collected after allowing differentiation of pluripotent stem cells such as iPS cells or ES cells derived from a donor.

### (Separation of Fibroblasts)

Typically, the fibroblasts prepared can be separated by treatment with an enzyme such as dispase or collagenase. The separation may be carried out by the enzyme such as dispase or collagenase, or may be carried out by another treatment such as mechanical treatment as long as the treatment enables the necessary separation.

### (Method of Producing VCAM1⁺ and CD90⁺ Fibroblasts)

The method comprises: bringing TNF-α and IL-4 into contact with fibroblasts; and culturing the contacted fibroblasts under conditions where both VCAM1 and CD90 can become positive.

The method of bringing TNF-α and IL-4 into contact with the fibroblasts is not limited as long as the induction of the VCAM1⁺ and CD90⁺ fibroblasts is not remarkably inhibited. The method is typically, but not limited to, addition of TNF-α and IL-4 to the medium containing the fibroblasts. Each of the TNF-α and IL-4 added may be of a single type, or may be of a plurality of types. In cases where the TNF-α or IL-4 is of a plurality of types, the plurality of types may be brought into contact with the fibroblast either separately or at the same time. For example, TNF-α and IL-4 may be dissolved in water, a medium, serum, or dimethyl sulfoxide (DMSO), and then the resulting solution may be added to the medium.

The fibroblasts can be cultured in a medium supplemented with TNF-α and IL-4. Therefore, the fibroblasts can be cultured while the VCAM1 and CD90 expression levels in the fibroblasts are maintained. Thus, fibroblasts having high VCAM1 and CD90 expression levels can be produced.

In cases where TNF-α and IL-4 are added to the medium (mL), the amount of the TNF-α added is not limited. The amount is usually not less than 0.1 ng/mL, may be not less than 0.5 ng/mL, may be not less than 1 ng/mL, may be not less than 10 ng/mL, or may be not less than 50 ng/mL. There is no upper limit of the amount, and the amount is usually not more than 500 ng/mL, or may be not more than 100 ng/mL.

The amount of the IL-4 added is not limited. The amount is usually not less than 0.1 ng/mL, may be not less than 0.5 ng/mL, may be not less than 1 ng/mL, or may be not less than 2 ng/mL. There is no upper limit of the amount, and the amount is usually not more than 10 ng/mL, may be not more than 5 ng/mL, or may be not more than 1 ng/mL.

The ratio (weight ratio) between the TNF-α and the IL-4 added, in terms of TNF-α: IL-4, is usually 10,000:1 to 1:1, or may be 50,000:1 to 10:1. Further, the ratio is usually 1:1 to 1:10,000, or may be 1:10 to 1:50,000.

By further culturing the fibroblasts after the contact with TNF-α and IL-4, VCAM1⁺ and CD90⁺ fibroblasts can be produced.

The conditions for the culture of the fibroblasts are not limited as long as the fibroblasts become positive for VCAM1 and CD90, and the culture may be carried out by a known cell culture method.

The medium used for the culture may be appropriately set according to, for example, the type of the cells to be cultured. For example, DMEM, α-MEM, RPMI-1640, HFDM-1(+), or the like may be used. The medium may be supplemented with nutritional substances such as FCS and FBS; growth factors; cytokines; antibiotics; and the like. Further, the culture may be carried out in a medium containing TNF-α and IL-4.

The number of hours or number of days of the culture period may be appropriately set depending on the purpose. For example, the culture may be carried out until a desired number of cultured cells are obtained, and/or until the cultured cells acquire a desired function. Examples of the culture period include periods such as 1 hour, 2 hours, 3 hours, 4 hours, 5 hours, 6 hours, 7 hours, 8 hours, 9 hours, 10 hours, 11 hours, 12 hours, 15 hours, 18 hours, 21 hours, 1 day, 2 days, 3 days, 4 days, 5 days, 6 days, 7 days, 8 days, 9 days, 10 days, 2 weeks, 1 month, 2 months, 3 months, and 6 months.

The culture temperature may be appropriately set according to the type of the cells to be cultured. The culture temperature may be, for example, not less than 10°C, not less than 15°C, not less than 20°C, not less than 25°C, or not less than 30°C, and may be, for example, not more than 60°C, not more than 55°C, not more than 50°C, not more than 45°C, or not more than 40°C.

The culture may be carried out a plurality of times. For example, the culture may be carried out each time when the purity of the desired fibroblasts is increased by selection or concentration.

The production of the fibroblasts may also include selection or concentration of VCAM1⁺ and CD90⁺ fibroblasts using an anti-CD106 antibody and/or anti-CD90 antibody. The method of the selection or concentration is not limited, and the selection or concentration can be carried out by a method known to those skilled in the art. The selection or concentration can be carried out, for example, by performing primary immunostaining with human CD106 (VCAM-1)-biotin (Miltenyi Biotec), performing secondary immunostaining with anti-biotin microbeads (Miltenyi Biotec), and then collecting the stained cells with autoMACS (Miltenyi Biotec); or by performing primary immunostaining with CD90-PE antibody (Miltenyi Biotec), performing secondary immunostaining with anti-PE microbeads (Miltenyi Biotec), and then collecting the stained cells with autoMACS (Miltenyi Biotec). The selection or concentration may be carried out at any timing.

As the anti-CD106 antibody, a known antibody can be used, and a commercially available product can be obtained and used.

Regarding the collection of the cultured fibroblasts, for example, the fibroblasts may be detached by a protease such as trypsin, and then collected. Alternatively, a temperature-responsive culture dish may be used. In this case, the fibroblasts may be detached by changing the temperature, and then collected. Alternatively, the fibroblasts may be detached using a tool such as a cell scraper, and then collected.

By administering or implanting the pharmaceutical composition of the present embodiment into a living body, a kidney disease can be treated. Examples of the kidney disease include, but are not limited to, acute kidney injury (AKI), chronic kidney disease (CKD), diabetic nephropathy, acute glomerulonephritis (acute nephritis), chronic glomerulonephritis (chronic nephritis), acute progressive glomerulonephritis, nephrotic syndrome, acute renal failure, and chronic renal failure. The kidney injury is preferably acute kidney injury (AKI) or chronic kidney disease (CKD).

The method of administering or implanting the pharmaceutical composition of the present embodiment into a living body is not limited. For example, the pharmaceutical composition can be administered by injection. By directly administering or implanting the pharmaceutical composition into a kidney tissue and/or a surrounding area thereof (such as beneath the renal capsule), treatment of the above-described kidney disease is possible. The pharmaceutical composition may be administered into the vein, artery, lymph node, lymphatic vessel, bone marrow, subcutaneous tissue, corpus cavernosum penis, or abdominal cavity. The administration into the the vein, artery, or lymphatic vessel may be carried out by injection into the vessel, infusion via a catheter, or another known method.

The method of the injection is not limited, and a known injection method such as needle injection or needle-free injection may be applied. The method of the infusion via a catheter is also not limited, and a known method may be applied.

The pharmaceutical composition of the present embodiment may be, but does not necessarily need to be, applied as an artificial organ material to a living body. The artificial organ material may be provided, for example, as a planar or three-dimensional cellular tissue constructed with cells. The planar or three-dimensional cellular tissue may be prepared by culturing the fibroblasts alone, or may be prepared by co-culturing the fibroblasts with other cells such as kidney cells and then forming the co-cultured cells into the planar or three-dimensional cellular tissue. Examples of the planar or three-dimensional cellular tissue include, but are not limited to, cell sheets and cell fibers; and tissues formed by a 3D printer.

In the present embodiment, the size and shape of the planar or three-dimensional cellular tissue are not limited as long as the tissue can be applied to a necrotic, damaged, or fibrotic kidney tissue, and/or to a surrounding area and/or a lesion area thereof. The planar tissue may be either a monolayer tissue or multilayer tissue.

The conditions for the cell culture, such as the medium composition, temperature, and humidity, are not limited as long as the VCAM1⁺ and CD90⁺ fibroblasts can be cultured, and as long as the planar or three-dimensional cellular tissue can be constructed.

By application of such a planar or three-dimensional cellular tissue to a necrotic, damaged, or fibrotic kidney tissue and/or a surrounding area thereof, or by using such a planar or three-dimensional cellular tissue as an artificial organ to replace a necrotic, damaged, or fibrotic kidney tissue and/or a surrounding area thereof, a kidney disease can be treated.

The ratio of the VCAM1⁺ and CD90⁺ fibroblasts contained in the pharmaceutical composition is not limited as long as the ratio is appropriately set based on, for example, the mode of administration or implantation into the patient, and as long as the composition comprises a therapeutically effective amount of the VCAM1⁺ and CD90⁺ fibroblasts as an effective component.

The number of the VCAM1⁺ and CD90⁺ fibroblasts contained in the pharmaceutical composition may be, for example, not less than 1.0×10² cells/mL, not less than 1.0×10³ cells/mL, not less than 1.0×10⁴ cells/mL, not less than 1.0×10⁵ cells/mL, not less than 5.0×10⁶ cells/mL, or not less than 1.0×10⁷ cells/mL. The number of the VCAM1-positive fibroblasts contained in the injectable composition may be further increased or decreased depending on the severity of the disease.

The method of confirming the therapeutic effect of the pharmaceutical composition is not limited, and examples of the method include confirmation of improvement of the renal function based on comparison between the renal function before the administration or implantation of the pharmaceutical composition and the renal function after the administration or implantation of the pharmaceutical composition. The improvement of the renal function can be judged, for example, based on the fact that the urine output, which had increased due to the kidney disease, shows a decrease toward a normal urine output after the administration of the pharmaceutical composition, or the fact that the serum creatinine level (Cre) or blood urea nitrogen level (BUN), which had been elevated due to the kidney disease, shows a decrease toward a normal concentration range after the administration of the pharmaceutical composition.

The pharmaceutical composition may also contain other components that are physiologically acceptable in the pharmaceutical composition. Examples of such other components include physiological saline, cell preservation liquids, cell culture media, hydrogels, extracellular matrices, and cryopreservation liquids.

Another embodiment of the present invention may be a therapeutic agent for a kidney disease, the agent consisting of cardiac fibroblasts. The agent is characteristic in that the cardiac fibroblasts are used for treatment of the kidney disease.

In the present embodiment, the cardiac fibroblasts may contain VCAM1⁺ and CD90⁺ fibroblasts. Although the ratio of the VCAM1⁺ and CD90⁺ fibroblasts to the total fibroblasts in the therapeutic agent is not limited, the ratio may be, for example, not less than 0.82%, may be not less than 1.08%, may be not less than 5%, may be not less than 10%, may be not less than 20%, may be not less than 30%, may be not less than 40%, may be not less than 49.07%, may be not less than 50%, may be not less than 60%, may be not less than 70%, may be not less than 71.96%, may be not less than 79.75%, may be not less than 80%, may be not less than 89.89%, may be not less than 90%, or may be not less than 95%; or may be more than 0.82%, may be more than 1.08%, may be more than 5%, may be more than 10%, may be more than 20%, may be more than 30%, may be more than 40%, may be more than 49.07%, may be more than 50%, may be more than 60%, may be more than 70%, may be more than 71.96%, may be more than 79.75%, may be more than 80%, may be more than 89.89%, may be more than 90%, or may be more than 95%; in terms of the cell number.

In the present embodiment, the cardiac fibroblasts may contain VCAM1⁺ fibroblasts. Although the ratio of the VCAM1⁺ fibroblasts in the human-derived fibroblasts is not limited, the ratio may be, for example, not less than 1.18%, may be not less than 1.53%, may be not less than 5%, may be not less than 10%, may be not less than 20%, may be not less than 30%, may be not less than 40%, may be not less than 50%, may be not less than 60%, may be not less than 70%, may be not less than 80%, may be not less than 80.86%, may be not less than 90%, may be not less than 90.28%, may be not less than 90.91%, may be not less than 93.49%, or may be not less than 95%; or may be more than 1.18%, may be more than 1.53%, may be more than 5%, may be more than 10%, may be more than 20%, may be more than 30%, may be more than 40%, may be more than 50%, may be more than 60%, may be more than 70%, may be more than 80%, may be more than 80.86%, may be more than 90%, may be more than 90.28%, may be more than 90.91%, may be more than 93.49%, or may be more than 95%; in terms of the cell number.

In the present embodiment, the cardiac fibroblasts may contain CD90⁺ fibroblasts. Although the ratio of the CD90⁺ fibroblasts in the human-derived fibroblasts is not limited, the ratio may be, for example, not less than 41.55%, may be not less than 50%, may be not less than 55.17%, may be not less than 56.83%, may be not less than 60%, may be not less than 70%, may be not less than 76.83%, may be not less than 80%, may be not less than 86.49%, may be not less than 90%, may be not less than 94.58%, or may be not less than 95%; or may be more than 41.55%, may be more than 50%, may be more than 55.17%, may be more than 56.83%, may be more than 60%, may be more than 70%, may be more than 76.83%, may be more than 80%, may be more than 86.49%, may be more than 90%, may be more than 94.58%, or may be more than 95%; in terms of the cell number.

The fibroblasts in the therapeutic agent of the present embodiment may be a fibroblast population containing the VCAM1⁺ and CD90⁺ fibroblasts described above.

In cases where the cardiac fibroblasts contain VCAM1⁺ and CD90⁺ fibroblasts in the present embodiment, the cardiac fibroblasts can be produced by the production method described in the section of (Method of Producing VCAM1⁺ and CD90⁺ Fibroblasts) described above for the pharmaceutical composition for treatment of a kidney disease.

The descriptions in the sections of (Preparation of Fibroblasts) and (Separation of Fibroblasts), the type of the kidney disease, the administration or implantation method, the number of fibroblasts, the method of confirming the therapeutic effect, and the like described above for the pharmaceutical composition for treatment of a kidney disease are applicable to the present embodiment.

In the present embodiment, the term "consisting of cardiac fibroblasts" encompasses inclusion of other components that are physiologically acceptable in the therapeutic agent, in addition to the cardiac fibroblasts. Examples of such other components include physiological saline, cell preservation liquids, cell culture media, hydrogels, extracellular matrices, and cryopreservation liquids.

Another embodiment of the present invention may be a method of treating a kidney disease by administering or implanting the pharmaceutical composition comprising VCAM1⁺ and CD90⁺ fibroblasts to a kidney tissue and/or a surrounding area thereof.

Still another embodiment of the present invention may be use of the pharmaceutical composition comprising VCAM1⁺ and CD90⁺ fibroblasts, as an injection solution or implant to be administered or implanted to a kidney tissue and/or a surrounding area thereof, for treatment of a kidney disease.

### EXAMPLES

The present invention is described below in more detail by way of Examples. Needless to say, however, the scope of the present invention is not limited to the Examples.

### isolation, Expansion, and Cell Sorting of Fibroblasts>

First, for isolating human adult cardiac fibroblasts (CFs), the whole heart of a human adult was purchased (Science Care, Phoenix, AZ). Subsequently, five pieces of a fresh human myocardial tissue, each having a size of about 2 mm², was obtained from the right auricle. Thereafter, the myocardial tissue was washed with phosphate buffered saline (PBS), and subjected to enzymatic digestion with 500 U/mL of liberase (Roche, Basel, Switzerland) for 1 hour. The digested tissue was cultured on a T25 flask using HFDM-1(+) medium (Cell Science & Technology Institute, Inc., Miyagi, Japan) supplemented with 20% (v/v) Newborn Calf Serum (NBCS). Ten days after the plating, the CFs were subcultured in another cell culture flask, and expanded by culturing in HFDM-1(+) medium supplemented with 1% (v/v) NBCS. The expanded CFs were used for the following experiment.

In order to isolate VCAM1⁺ and CD90⁺ CFs, the CFs were cultured with 50 ng/mL TNF-α (PeproTech, Cranbury, NJ) and 2 ng/mL IL-4 (FUJIFILM Wako Pure Chemical Corporation, Osaka, Japan), to upregulate the expression of VCAM1 on the CFs as previously reported (Patent Document 1). Thereafter, labeling with CD90-PE antibody (Miltenyi Biotec, Bergisch Gladbach, Germany) and anti-PE microbeads (Miltenyi Biotec) was carried out, and CD90⁺ CFs were isolated from the uCFs using CD90 as an index by an autoMACS Pro Separator (Miltenyi Biotec, Bergisch Gladbach, Germany).

### <Flow Cytometry Analysis>

The CFs, uCFs, and u90⁺ CFs were incubated with CD90-PE antibody (Miltenyi Biotec) and CD106 (VCAM1)-BV421 antibody (BD Biosciences, San Jose, CA) for 30 minutes. REA control(S)-PE (Miltenyi Biotec) and Mouse IgG1, κ Isotype Control-BV421 (BD Biosciences) were used as isotype controls. FCM analysis was carried out using a MACSQuant Analyzer in accordance with the manufacturer's instructions (Miltenyi Biotec).

### <Animal Experiment>

The animal experiment in Example 2 was carried out with approval by the animal experiment committee of Tokyo Women's Medical University (approval number: AE21-017). Male athymic nude rats (F344/NJcl-rnu/mu) were purchased at 8 to 9 weeks old (CLEA Japan, Inc., Tokyo, Japan), and maintained until 10 to 11 weeks old, which corresponds to a body weight of 200 to 250 g. Every effort was made in order to minimize the pain of the animals. All animal experiments were carried out in a blinded manner.

The rats were anesthetized with 3 to 4% isoflurane using a vaporizer, and 1.5 to 2% isoflurane was maintained on a heating plate kept at 37°C during the surgery. No analgesic was used, but the incision site was minimized. All rats were subjected to right nephrectomy 1 week before the ischemic reperfusion injury (IRI) treatment. The right kidney was exposed by laparotomy. The renal artery, renal vein, and ureter were carefully separated from the surrounding fat, and then ligated and excised, to remove the right kidney. On Day 7 after the right nephrectomy, the rats were subjected to midline incision to expose the left kidney, left renal artery, and left renal vein. The renal pedicle was then clamped with a disposable vascular clip (TKM-1; Bear Medic Corporation, Tokyo, Japan) for 40 minutes. The clip was removed, and reperfusion was visually confirmed. Thereafter, cells or a vehicle was/were injected immediately beneath the renal capsule as described below.

The animals were randomly divided into three groups, and different test substances were administered to these groups: injection of human CFs (CFs: addition of 3×10⁶ cells to 50 µL of Bambanker (Nippon Genetics Co., Ltd., Tokyo, Japan); n=7); injection of human VCAM1⁺ CFs (uCFs: addition of 3×10⁶ cells to 50 µL of Bambanker; n=2); injection of human VCAM1⁺ and CD90⁺ CFs (u90⁺ CFs: addition of 3×10⁶ cells to 50 µL of Bambanker; n=4); and injection of a vehicle (control group: 50 µL of Bambanker; n=7). The renal function was monitored in terms of serum creatinine (Cre) and blood urea nitrogen (BUN). Blood samples were collected from the tail vein on Day -2, Day 2, Day 4, Day 7, and Day 14 as counted from the day of cell administration (Day 0). Urine was collected on Day -2, Day 4, and Day 14 as counted from the day of cell administration (Day 0). The body weight was monitored on Day - 7, Day -2, Day 0, Day 2, Day 4, Day 7, and Day 14 as counted from the day of cell administration (Day 0). At the end of the monitoring period (Day 14), the animals were sacrificed. The left kidney was removed, weighed, and then fixed with 4% paraformaldehyde (Muto Pure Chemicals Co., Ltd., Tokyo, Japan). The animals for which the IRI treatment failed, and the animals which died immediately after the operation were excluded from the test. The rats which died during the postoperative observation period were included in the analysis of the survival rate, but excluded from other experiments.

The animal experiment in Example 3 was carried out with approval by the animal experiment committee of Tokyo Women's Medical University (approval number: AE22-002). Male SD rats were purchased at 9 weeks old (Japan SLC, Inc., Shizuoka, Japan). The experiment was carried out after several days of acclimation. Every effort was made in order to minimize the pain of the animals.

The SD rats were anesthetized, and the right ureter was carefully separated and ligated. After the ureteral ligation, the test substance was topically administered immediately beneath the renal capsule in all animals.

The animals were randomly divided into four groups, and different test substances were administered to these groups: injection of a vehicle (control group: 70 µL of physiological saline supplemented with 0.33% hyaluronic acid, n=2); injection of CFs (CFs, addition of 3×10⁶ cells to 70 µL of the vehicle, n=1); injection of CFs treated with TNF-α and IL-4 (uCFs, addition of 3×10⁶ cells to 70 µL of the vehicle, n=1); and injection of VCAM1⁺ and CD90⁺ CFs (u90⁺ CFs, addition of 3×10⁶ cells to 70 µL of the vehicle, n=2). The animals were sacrificed 1 week after the administration of the test substance, followed by collection of kidneys.

### <Tissue Staining>

The tissue staining in Example 2 was carried out as follows. After the removal of the kidneys, the kidneys were fixed, and embedded in paraffin. For histological analysis, tissues were sliced at a thickness of 10 µm, and stained with Sirius Red. Interstitial fibrosis of areas positive for the Sirius Red staining was quantified by ImageJ/FIJI software.

The tissue staining in Example 3 was carried out as follows. After the removal of the kidneys, the kidneys were fixed, and embedded in paraffin. For histological analysis, tissues were sliced at a thickness of 5 µm, and stained with Sirius Red or PAS (Periodic acid Schiff).

<Example 1: Characteristics of Primary Cultured Cardiac Fibroblasts Derived from Human Adult>

Human cardiac fibroblasts (CFs) were isolated from a human myocardial tissue collected from the right auricle of a whole heart, and cultured. As a result, the CFs were elongated and proliferated to exhibit a flat spindle shape, which is typical as a phenotype of fibroblasts (Fig. 1A). As a result of flow cytometry analysis, the CFs were found to be expressing VCAM1 at 1.07 ± 0.46% and CD90 at 46.11 ± 9.06%, and the ratio of their double-positive (DP) was found to be 0.74 ± 0.34% (n=2, mean ± standard deviation (SD), Fig. 1B). In order to improve the expression of VCAM1 and CD90 in the CFs, the CFs were treated with 50 ng/mL TNF-α and 2 ng/mL IL-4 for 3 days as previously reported (Patent Document 1). This treatment resulted in improved expression of both VCAM1 and CD90. The CFs (uCFs) cultured with TNF-α and IL-4 were found to be expressing VCAM1 at 90.91% and CD90 at 76.83%, and the ratio of DP was 71.96% (n=1; Fig. 1B). Subsequently, VCAM1⁺ and CD90⁺ CFs were isolated from the uCFs (u90⁺ CFs). As a result, VCAM1 was found to be expressed at 95.43 ± 1.94%, and CD90 was found to be expressed at 97.47 ± 2.89%. The ratio of DP was 93.57 ± 3.68% (n=6, mean ± SD; Fig. 1B).

### <Example 2: uCF and u90⁺ CFs Improve Renal Function in Rat Model with AKI Induced by IRI>

In order to investigate whether uCF and u90⁺ CFs improve the renal function in a rat model with AKI induced by IRI, CFs, uCFs, or u90⁺ CFs were administered to this rat model. An outline of the experimental procedure is shown in Fig. 2A. Comparison was made among the cell administration groups and the vehicle control group (represented as Ctrl in the figure). As a result, the uCF administration group (represented as +uCFs in the figure), the u90⁺ CF administration group (represented as +u90⁺ CFs in the figure), and the vehicle control group showed higher survival rates than the survival rate of the CF administration group (represented as +CFs in the figure) (Fig. 2B). The body weight decreased in the CF administration group and the uCF administration group, and similar tendencies were found in the vehicle control group and the u90⁺ CF administration group (Fig. 2C). On the other hand, the mean kidney weight was the lowest in the +u90⁺ CF administration group (Fig. 2D), and the urine output was the highest in the vehicle control group on Day 4 after the cell administration (Fig. 2E). Regarding the renal function, the values of Cre and BUN in the CF, uCF, or u90⁺ CF administration group decreased on Days 2 to 7 after the cell administration (Figs. 2F, 2G, 2H, 2I, 2J, and 2K).

Further, by SR staining, the presence of a fibrotic structure in the kidney tissue in each group was revealed (Fig. 3A). The degree of fibrosis (that is, the ratios of the SR-stained surface area in the central part, the dorsal and ventral part, and the whole kidney) tended to decrease in the CF administration group or the u90⁺ CF administration group (Fig. 3B). It was thus found that administration of uCFs or u90⁺ CFs to a subject with AKI induced by IRI leads to a high survival rate, and improves the renal function. Further, u90⁺ CFs were also found to be effective for suppressing fibrosis.

### <Example 3: Fibrosis-Preventing/Improving Effect of CD90⁺ and VCAM1⁺ Human Cardiac Fibroblasts in Renal Fibrosis Rat Model>

Since a kidney disease hardly causes subjective symptoms in the early stage, some of the subject patients in the present invention are patients with chronic kidney disease (CKD) exhibiting advanced remodeling. In view of this, the present inventors focused on a therapeutic effect on fibrosis, which is the main reaction of renal remodeling in CKD, and aimed to confirm the effect of adult cardiac fibroblasts on CKD. More specifically, adult cardiac fibroblasts were injected into a unilateral ureteral obstruction (UUO) model, which is widely used as a renal fibrosis model, and a therapeutic effect and a suppressive effect on renal fibrosis were evaluated from a pathological perspective.

First, characteristics of primary human adult cardiac fibroblasts were investigated. Human cardiac fibroblasts (CFs) were isolated from a human myocardial tissue collected from the right auricle of a heart, and cultured. As a result, the CFs were elongated and proliferated to exhibit a flat spindle shape, which is typical as the cell morphology of fibroblasts (Fig. 4A). Results of flow cytometry analysis are shown in Fig. 4B. The CFs were found to be expressing VCAM1 at 1.18% and CD90 at 41.55%, and the ratio of their double-positive (DP) was found to be 0.82% (n=1). The CFs treated with TNF-α and IL-4 (uCFs) were found to be expressing VCAM1 at 80.86% and CD90 at 56.83%, and the ratio of their double-positive (DP) was found to be 49.07% (n=1). Subsequently, the uCFs were subjected to cell sorting using CD90 as an index, and a positive fraction was collected. As a result, the u90⁺ CFs, obtained by isolation of VCAM1⁺ and CD90⁺ CFs, were found to be expressing VCAM1 at 90.28% and CD90 at 86.49%, and the ratio of their double-positive (DP) was found to be 79.75% (n=1).

In order to evaluate the effectiveness of CD90⁺ and VCAM1⁺ human adult cardiac fibroblasts on renal fibrosis, first, a UUO rat model was prepared by surgical operation, and various cardiac fibroblasts (CFs, uCFs, or u90⁺ CFs) were administered thereto. One week after the cell administration, kidneys were collected, and the cortical region and the medullary outer layer region in each group were observed by Sirius Red staining and PAS staining. The results are shown in Fig. 5. In Fig. 5, Ctrl represents stained images in a vehicle control group, and Sham represents stained images of left kidney tissues without ureteral ligation. As a result of the Sirius Red staining, Sham and the u90⁺ CF administration group hardly exhibited fibrosis in either the cortical region or the medullary outer layer region. However, the other groups exhibited extensive fibrotic areas. Further, in the PAS staining, Sham and the u90⁺ CF administration group exhibited only low degrees of proximal tubular damage and tubular dilation in both the cortical region and the medullary outer layer region. However, in the other groups, proximal tubular damage and tubular dilation were extensively found, and there were findings of tubular atrophy and necrosis. In all groups except Sham and the u90⁺ CF administration group, the brush border was found to be detached and hardly remaining.

From these results, it was shown that u90⁺ CFs contribute to the prevention and treatment of renal fibrosis, and that they produce a beneficial effect on kidney tissue structures associated with renal injury. It was thus suggested that u90⁺ CFs are effective not only for fibrosis, but also for renal functions due to structural defects associated with exacerbation of chronic kidney disease.

## Claims

1. A pharmaceutical composition for treatment of a kidney disease, the composition comprising VCAM1⁺ and CD90⁺ fibroblasts,
wherein the ratio of the VCAM1⁺ and CD90⁺ fibroblasts in the total fibroblasts in the pharmaceutical composition is more than 1.08%.

2. The pharmaceutical composition according to claim 1, wherein the fibroblasts are cardiac fibroblasts.

3. The pharmaceutical composition according to claim 1 or 2, wherein the fibroblasts are derived from an adult.

4. The pharmaceutical composition according to any one of claims 1 to 3, wherein the kidney disease is acute kidney injury or chronic kidney disease.

5. The pharmaceutical composition according to any one of claims 1 to 4, which is an injectable composition.

6. The pharmaceutical composition according to any one of claims 1 to 4, wherein the fibroblasts are constructed as a planar or three-dimensional cellular tissue.

7. A therapeutic agent for a kidney disease, the agent comprising cardiac fibroblasts.

8. The therapeutic agent according to claim 7,
wherein the cardiac fibroblasts comprise VCAM1⁺ and CD90⁺ fibroblasts, and
wherein the ratio of the VCAM1⁺ and CD90⁺ fibroblasts in the total fibroblasts in the therapeutic agent is more than 1.08%.

9. The therapeutic agent according to claim 7 or 8, wherein the cardiac fibroblasts are derived from an adult.

10. The therapeutic agent according to any one of claims 7 to 9, wherein the kidney disease is acute kidney injury or chronic kidney disease.

11. The therapeutic agent according to any one of claims 7 to 10, which is an injectable composition.

12. The therapeutic agent according to any one of claims 7 to 10, wherein the fibroblasts are constructed as a planar or three-dimensional cellular tissue.
